(19) Europäisches Patentamt / European Patent Office / Office européen des brevets

(11) **EP 3 745 180 A1**

(12) **EUROPEAN PATENT APPLICATION**

(43) Date of publication:
**02.12.2020 Bulletin 2020/49**

(51) Int Cl.:
**G02B 21/00** (2006.01)     **G01N 21/64** (2006.01)
**G02B 21/08** (2006.01)

(21) Application number: **19199038.1**

(22) Date of filing: **23.09.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2019   TW 108118552**

(71) Applicant: **CAL-COMP BIG DATA, INC Shenkeng, New Taipei City 222 (TW)**

(72) Inventors:
• **HUANG, Yuan-Peng 222 NEW TAIPEI CITY (TW)**
• **WENG, Chu-Chiang 222 NEW TAIPEI CITY (TW)**

(74) Representative: **2K Patentanwälte Blasberg Kewitz & Reichel Partnerschaft mbB Schumannstrasse 27 60325 Frankfurt am Main (DE)**

Remarks:
Amended claims in accordance with Rule 137(2) EPC.

(54) **FLUORESCENT MICROSCOPIC IMAGING APPARATUS**

(57)     A fluorescent microscopic imaging apparatus (1) for detecting a skin to be tested includes a light guiding structure (10), a light emitting module (20), an imaging module (30) and an excitation light blocking filter unit (40). A first plane (11) of the light guiding structure (10) contacts the skin to be tested. The light emitting module (20) includes a plurality of white light emitting units (21) and a plurality of excitation light emitting units (22) those are staggered with each other. The imaging module (30) is disposed at a central position of the light emitting module (20). The excitation light blocking filter unit (40) blocks an excitation light with a specific wavelength emitted by each excitation light emitting unit (22).

FIG.1

## Description

## BACKGROUND OF THE INVENTION

### Technical Field

The present disclosure relates to a fluorescent microscopic imaging apparatus, and more particularly to a fluorescent microscopic imaging apparatus that may switch between a white light emitting unit and an excitation light emitting unit without interfering with an image received.

### Description of Related Art

The statements in this section merely provide background information related to the present disclosure and do not necessarily constitute prior art.

In the field of biomedicine, a fluorescence microscope may be applied to study about absorption, transportation, distribution and localization of chemical substances in cells. Certain chemicals in the cells, such as chlorophyll, are illuminated by ultraviolet light (UV) and excited to produce fluorescence. Other chemicals may not be excited to produce the fluorescence, but if they are dyed with fluorescent dyes or fluorescent antibodies, they may also be excited by ultraviolet light. Then, the fluorescence microscope is one of the tools for qualitative and quantitative research on such chemicals.

In the actual use of the fluorescent microscope needs to place an excitation filter before an excitation light source, and an emission filter needs to be placed in front of an imaging module for optical spectrum processing. The excitation filter only allows excitation light to pass. The emission filter only allows the fluorescence to pass. However, after the fluorescence is observed, it is necessary to remove the analyte from the fluorescence microscope, and it is impossible to immediately switch to use visible light as the light source in the same field of view for microscopic observation. (Visible light will be blocked by the emission filter). Especially for microscopic observation applications, in the process of replacing the light source, it is easy to cause confusion and misjudgment in the image comparison due to the deviation of the field of view, and it is impossible to accurately judge the state of the observation result.

Further, for a digital microscope using visible light as a light source, there is usually no requirement for the uniformity of light intensity (which is recognized by the naked eye). However, for the fluorescence microscopy, the intensity of observed fluorescence may be related to size, quantity or distribution of the target. In the interpretation of the actual measurement, a light intensity of the excitation light is not uniform and it is easy to cause a serious error in the final observation result, so the uniformity of the light intensity is very important.

Therefore, how to design fluorescent microscopic imaging apparatus to solve the technical problems above is an important subject studied by the inventors and proposed in the present disclosure.

## SUMMARY OF THE INVENTION

The purpose of the present disclosure is to provide a fluorescent microscopic imaging apparatus that may improve the uniformity of light intensity of the excitation light. After the fluorescence observation is completed, the fluorescent microscopic imaging apparatus may immediately switch to use visible light as the light source in the same field of view for microscopic observation, thereby achieving the purpose of convenient to use and accurate to judgment.

In order to achieve the objective, the fluorescent microscopic imaging apparatus for detecting a skin to be tested including a light guiding structure, a light emitting module, an imaging module and an excitation light blocking filter unit. The light guiding structure having a first plane and a second plane, the first plane contacts the skin to be tested. The light emitting module is disposed on the second plane, the light emitting module includes a plurality of white light emitting units and a plurality of excitation light emitting units staggered with each other. The imaging module is disposed on the second plane and disposed at a central position of the light emitting module. The excitation light blocking filter unit is disposed between the first plane and the imaging module, the excitation light blocking filter unit blocks an excitation light with a specific wavelength emitted by each excitation light emitting unit.

Further, the distance between the imaging module and the first plane is L, the distance between the imaging module and one side of each white light emitting unit is R, the distance between the imaging module and one side of each excitation light emitting unit is R, the minimum angle between a normal line of the first plane and an extension line of one side of each white light emitting unit is $a$, the minimum angle between the normal line of the first plane and an extension line of one side of each excitation light emitting unit is $a$, and the foregoing content satisfies the following relationship:

$$\tan a = \frac{R}{L}, a \geq 30°.$$

Further, the light guiding structure is a frustum of a cone, an annular surface that is opaque is formed between one side of the first plane and one side of the second plane, and an area of the first plane is smaller than an area of the second plane.

Further, the annular surface is covered with a reflective film.

Further, the reflective film is an aluminum metal film.

Further, the specific wavelength is between 350 nm and 400 nm.

Further, the specific wavelength is 365 nm.

Further, the excitation light blocking filter unit is covered with the imaging module.

Further, the excitation light blocking filter unit does not contact the imaging module.

Further, the excitation light blocking filter unit blocks light having a wavelength between 300 nm and 400 nm.

Further, the first plane is a flat glass covered with an anti-reflection film.

Further, the anti-reflection film provides a transmittance of light having a wavelength between 350 nm and 700 nm that is greater than 97%.

Further, the first plane is a flat glass covered with an oil-repellent hydrophobic film. Further, the imaging module includes a microscope lens, a photosensitive element and a control circuit board, the microscope lens is disposed in the second plane, and the microscope lens is disposed between the excitation light blocking filter unit and the photosensitive element, the photosensitive element and the control circuit board are disposed outside the second plane, and the photosensitive element is aligned with the microscope lens and coupled to the control circuit board.

Further, a distance between the microscope lens and each white light emitting unit is equal to a distance between the microscope lens and each excitation light emitting unit.

Further, the plurality of white light emitting units and the plurality of excitation light emitting units are arranged in a ring shape.

Further, the plurality of white light emitting units do not emit light simultaneously with the plurality of excitation light emitting units.

When the aforementioned fluorescent microscopic imaging apparatus of the present disclosure is used, it may improve the uniformity of light intensity of the excitation light by the relationship between the first plane of the light guiding structure, the imaging module, the plurality of white light emitting units and the plurality of excitation light emitting units of the light emitting module. Moreover, the excitation light blocking filter unit blocks only the excitation light having the specific wavelength, and allows another light than the specific wavelength to pass. The excitation light blocking filter unit is used together with the plurality of white light emitting units and the plurality of excitation light emitting units which are staggered with each other. After the fluorescence observation is completed, the fluorescent microscopic imaging apparatus may immediately switch to use visible light as the light source in the same field of view for microscopic observation, thereby achieving the purpose of convenient to use and accurate judgment. It will not cause confusion and misjudgment in the image comparison due to the replacement of the light source, thereby achieving the purpose of convenient to use and accurate to judgment.

In order to further understand the techniques, means, and effects of the present disclosure for achieving the intended purpose. Please refer to the following detailed description and drawings of the present disclosure. The drawings are provided for reference and description only, and are not intended to limit the present disclosure.

BRIEF DESCRIPTION OF DRAWINGS

FIG. 1 is an external view of a fluorescent microscopic imaging apparatus of the present disclosure.
FIG. 2A is an exploded view of a first embodiment of the fluorescent microscopic imaging apparatus of the present disclosure.
FIG. 2B is an exploded view of a second embodiment of the fluorescent microscopic imaging apparatus of the present disclosure.
FIG. 3 is a cross-sectional view of the second embodiment of the fluorescent microscopic imaging apparatus of the present disclosure.
FIG. 4 is a function block diagram showing the electrical function of the fluorescent microscopic imaging apparatus of the present disclosure.

DETAILED DESCRIPTION OF THE INVENTION

The embodiments of the present disclosure are described by way of specific examples, and those skilled in the art can readily appreciate the other advantages and functions of the present disclosure. The present disclosure may be embodied or applied in various other specific embodiments, and various modifications and changes can be made without departing from the spirit and scope of the present disclosure.

It should be understood that the structures, the proportions, the sizes, the number of components, and the like in the drawings are only used to cope with the contents disclosed in the specification for understanding and reading by those skilled in the art, and it is not intended to limit the conditions that can be implemented in the present disclosure, and thus is not technically significant. Any modification of the structure, the change of the proportional relationship, or the adjustment of the size, should be within the scope of the technical contents disclosed by the present disclosure without affecting the effects and the achievable effects of the present disclosure.

The technical content and detailed description of the present disclosure will be described below in conjunction with the drawings.

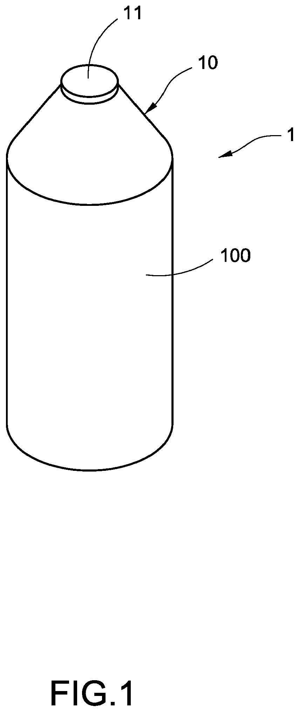

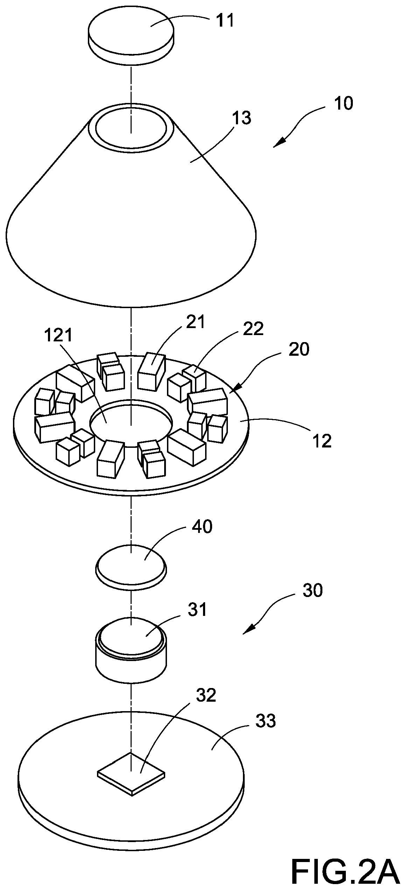

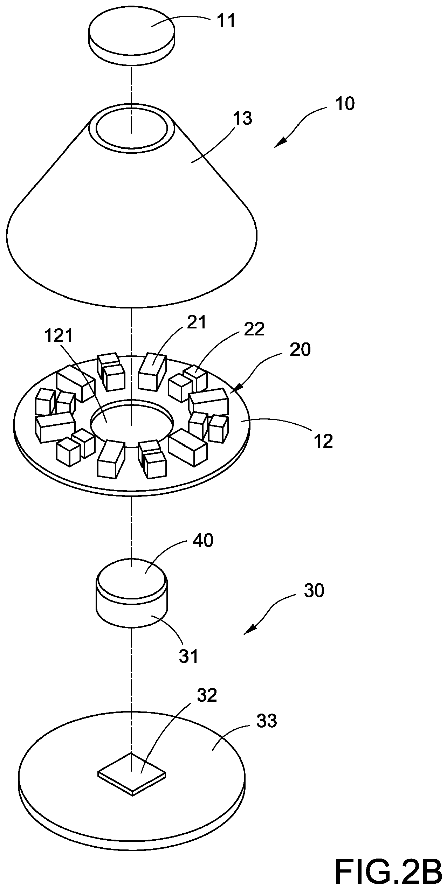

Please refer to FIG. 1 and FIG. 2, FIG. 1 is an external view of a fluorescent microscopic imaging apparatus of the present disclosure, FIG. 2A is an exploded view of a first embodiment of the fluorescent microscopic imaging apparatus of the present disclosure.

The fluorescent microscopic imaging apparatus 1 for detecting a skin to be tested (not shown) includes a light guiding structure 10, a light emitting module 20, an imaging module 30 and an excitation light blocking filter unit 40. The fluorescent microscopic imaging apparatus 1 of the present disclosure is a pen shape portable device, but the present disclosure is not limited thereto. The light guiding structure 10 is placed in a front section of the fluorescent microscopic imaging apparatus 1 for contacting the skin to be tested. A grip portion 100 is placed in a rear section of the fluorescent microscopic imaging apparatus 1 for the user to hold and carry. The light emitting module 20 and the excitation light blocking filter unit 40 are housed in the light guiding structure 10, and the imaging module 30 is housed in the light guiding structure 10 and the grip portion 100.

The light guiding structure 10 has a first plane 11 and a second plane 12. The first plane 11 contacts the skin to be tested, which flattens and fixes the uneven skin or the curved skin to be fixed at a fixed focus position to overcome the problem of difficultly focusing images at a shallow depth of field (e.g., depth of field less than 1mm) by the imaging module 30. It does not cause a part of the image to be clear but another part of the image to be blurred, and therefore it is easy to use and output stable image quality. In the first embodiment of the present disclosure, the light guiding structure 10 is a frustum of a cone (as shown in FIG. 2A). An annular surface 13 that is opaque and is formed between one side of the first plane 11 and one side of the second plane 12, and an area of the first plane 11 is smaller than an area of the second plane 12. Further, an opening 121 is formed in the center of the second plane 12.

The light emitting module 20 is disposed on the second plane 12, and the light emitting module 20 includes a plurality of white light emitting units 21 and a plurality of excitation light emitting units 22 staggered with each other. A plurality of white light emitting units 21 and a plurality of excitation light emitting units 22 may be composed of light emitting diodes (LEDs), and a plurality of white light emitting units 21 and a plurality of excitation light emitting units 22 are arranged in a ring shape, but the present disclosure is not limited thereto. Each excitation light emitting unit 22 emits excitation light having a specific wavelength. In the first embodiment of the present disclosure, the specific wavelength is between 350 nm and 400 nm. In particular, the specific wavelength optimized is 365 nm (the tolerance is plus and minus 5 nm), and the specific wavelength belongs to ultraviolet light (UV).

As an example of propionibacterium acnes, which is prone to cause skin diseases, it is a group of bacteria commonly found in the pores of the skin surface and is one of the main causes of acne. The cleanliness of the pores on the skin also affects the chance of causing skin diseases, but the average pore size of the pores is only about 0.9 mm, which needs to be observed with a microscope. Generally, a common digital microscopic device using visible light as a main light source is difficult to effectively distinguish and observe the growth state of propionibacterium acnes in the pores and whether the propionibacterium acnes is effectively cleaned due to its wavelength limitation. For this purpose, a fluorescence microscope is required for the detection of propionibacterium acnes. The propionibacterium acnes produces a chemical called "porphyrin" in the metabolic process, and the chemical structure of the porphyrin may be excited by the ultraviolet light (the main absorption peak is 365 nm) to excite an orange fluorescence that belongs to visible light range (the main emission peak is 660 nm). Therefore, it is possible to indirectly know the growth of propionibacterium acnes in each skin region by observing the light intensity of the orange fluorescence, the size of the dot region of the orange fluorescence, and the dot distribution.

The imaging module 30 is disposed on the second plane 12 and disposed at a central position of the light emitting module 20. Further, the imaging module 30 includes a microscope lens 31, a photosensitive element 32, and a control circuit board 33. The microscope lens 31 passes through the opening 121 of the second plane 12 and is convexly disposed within the second plane 12. The microscope lens 31 is disposed between the excitation light blocking filter unit 40 and the photosensitive element 32. A distance between the microscope lens 31 and each white light emitting unit 21 is equal to a distance between the microscope lens 31 and each excitation light emitting unit 22. The photosensitive element 32 and the control circuit board 33 are disposed outside the second plane 12, and the photosensitive element 32 is aligned with the microscope lens 31 and coupled to the control circuit board 33. The photosensitive element 32 may be constituted by a CCD or a CMOS. The control circuit board 33 may be a circuit board including an image signal processor (ISP).

The excitation light blocking filter unit 40 is disposed between the first plane 11 and the imaging module 30, the excitation light blocking filter unit 40 blocks an excitation light with a specific wavelength emitted by each excitation light emitting unit 22. The excitation light blocking filter unit 40 is an excitation block filter that may not contact the imaging module 30, as shown in FIG. 2A. It is different from an excitation filter placed in front of a light source in a general fluorescent microscope and it allows only excitation light to pass. The excitation light blocking filter unit 40 disposed in front of the imaging module 30 of the present disclosure blocks only the excitation light, and the light emitting module 20 of the present disclosure is not disposed with a filter. Therefore, when a condition that the plurality of white light emitting units 21 do not emit light simultaneously with the plurality of excitation light emitting units 22 is satisfied, then switch the light source to visible light emitted by the white light emitting unit 21, the visible light may pass through the excitation light blocking filter unit 40 into the imaging module 30. In the first embodiment of the present disclosure, the excitation light blocking filter unit 40 blocks light having a wavelength between 300 nm and 400 nm, so that the transmittance of light having a wavelength from 300 nm to 400 nm is less than 5%, and a transmittance of light having a wavelength from 400 nm to 700 nm is greater than 97%. As a result, in a fluorescent mode (only the excitation light emitting unit 22 works), the orange fluorescent light that generated by the excitation light exposure on the skin to be tested may be sensed by the photosensitive element 32. In the white light mode (only the white light emitting unit 21 works), the skin image with the general visible light wavelength (400 nm to 700 nm) may also be detected by the photosensitive element 32. Thereby, the optical design shared by the two modes is achieved.

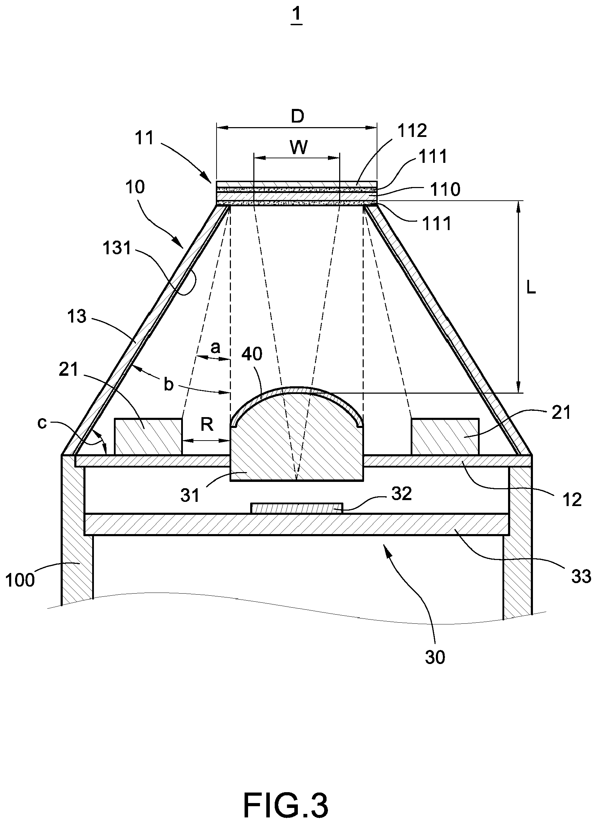

Please refer to FIG. 2B and FIG. 3, FIG. 2B is an exploded view of a second embodiment of the fluorescent microscopic imaging apparatus of the present disclosure, FIG. 3 is a cross-sectional view of the second embodiment of the fluorescent microscopic imaging apparatus of the present disclosure.

The second embodiment of the present disclosure is substantially the same as the first embodiment described above, except that the excitation light blocking filter unit 40 is covered with the imaging module 30. Further, the excitation light blocking filter unit 40 may be coated on the microscope lens 31 by physical vapor deposition (PVD) or chemical vapor deposition (CVD) to form a thin film. Further, the annealing or etching process may be performed after the film is formed to form a micro-lens structure to enhance the secondary optical effect, but the present disclosure is not limited thereto.

As shown in FIG. 3, the annular surface 13 is covered with a reflective film 131. The reflective film 131 may be an aluminum metal film having a reflectance is more than 90% for both visible light and ultraviolet light. The first plane 11 is a flat glass 110 covered with an anti-reflection film 111, and the anti-reflection film 111 provides a transmittance of light having a wavelength between 350 nm and 700 nm that is greater than 97%. It allows most of the visible light emitted by the white light emitting unit 21 to pass through the first plane 11, thereby reducing stray light caused by the visible light in the first plane 11 and preventing stray light from interfering with the photosensitive element 32 in FIG. 2B. The anti-reflection film 111 may be SiOx, SiNx or a sub-wavelength structure (SWC), but the present disclosure is not limited thereto. There is covered with an oil-repellent hydrophobic film 112 on one side of the flat glass 110 of the first plane 11 used to contact the skin to be tested, so as to prevent moisture or oil from the skin to be tested from entering the light guiding structure 10. The oil-repellent hydrophobic film 112 may be a lotus structure made by chemically surface modification or polymer material, but the present disclosure is not limited thereto. Further, the distance between the microscope lens 31 and the first plane 11 is L, which is the working distance of the microscope lens 31. The distance between the microscope lens 31 and one side of each white light emitting unit 21 is R, and the distance between the microscope lens 31 and one side of each excitation light emitting unit 22 is R (only the white light emitting unit 21 is shown in FIG.

3). The minimum angle between a normal line of the first plane 11 and an extension line of one side of each white light emitting unit 21 is *a*, the minimum angle between the normal line of the first plane 11 and an extension line of one side of each excitation light emitting unit 22 is *a* (only the white light emitting unit 21 is shown in FIG. 3), and the foregoing content satisfies the following relationship:

$$\tan a = \frac{R}{L}, \quad a \geq 30°, \ b < 45° \text{ and } c > 45°.$$

Wherein, the angle between the normal line of the first plane 11 and the annular surface 13 is b, the angle between the annular surface 13 and second plane 12 is c.

In the second embodiment of the present disclosure, the optimized embodiment corresponding to FIG. 3 may be as follows: L=15mm, *a*=30°, b=40°, c=50°, and R=8.66mm. It takes into account the positional and angular relationship of the annular surface 13 to the white light emitting unit 21 and the excitation light emitting unit 22, and the secondary optical effect. Under the condition of *a*≥30°, the relationship between the relative radiant intensity and the incident angle is linear, and the first plane 11 may have a better effect of light uniformization. Although the LEDs have different intensities at different divergence angles (0 to 90 degrees), the divergence angle range of 30 degrees to 70 degrees is a nearly linear change with respect to light intensity. Therefore, 30 degrees to 40 degrees is the best design interval in this disclosure. The arrangement position of the LEDs light out are made at an angle of 50 degrees to 70 degrees with respect to the light guiding structure 10 (for example, c=50°), and the LEDs are arranged in a circular symmetric manner on the second plane 12. Therefore, the light intensity of one side of the LEDs gradually decreasing due to the divergence angle is supplemented by the light intensity of the other side of the LEDs, so the light intensity is uniformized.

However, this design corresponds to a divergence angle in the interval of 0 to 30 degrees, where the angle at which the light intensity approaches a uniformity accumulates at the edge of the first plane 11 of the light guiding structure 10. Therefore, the following relative relationships need to be followed to conform to this design: The diameter of the first plane 11 of the light guiding structure 10 is D, and the diameter of the field of view (FOV) of the microscope lens 31 is W, where D is greater than W to avoid uneven light intensity in the field of view.

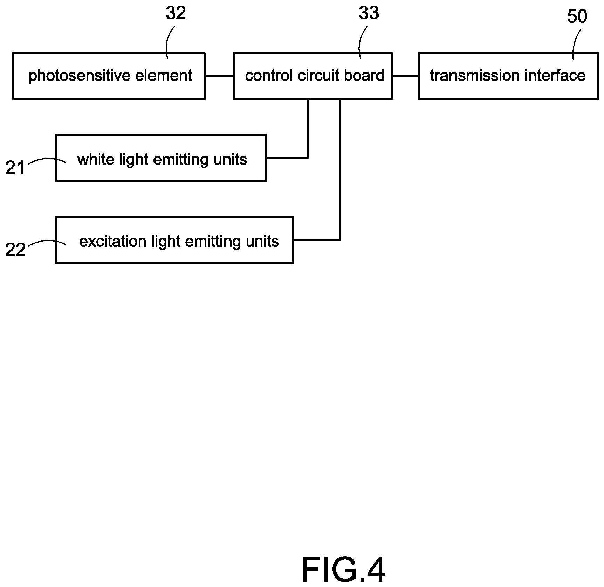

Please referring to FIG. 4, it is a function block diagram showing the electrical function of the fluorescent microscopic imaging apparatus of the present disclosure. The fluorescent microscopic imaging apparatus 1 of the present disclosure further includes a transmission interface 50. The transmission interface 50 is coupled to the control circuit board 33 for transmitting data related to the photosensitive element 32 on the control circuit board 33. The transmission interface 50 may be a USB port or a Wi-Fi communication unit.

When the aforementioned fluorescent microscopic imaging apparatus 1 of the present disclosure is used, it may improve the uniformity of light intensity of the excitation light by the relationship between the first plane 11 and the annular surface 13 of the light guiding structure 10, the imaging module 30, the plurality of white light emitting units 21 and the plurality of excitation light emitting units 22 of the light emitting module 20. Moreover, the excitation light blocking filter unit 40 blocks only the excitation light having the specific wavelength, and allows another light than the specific wavelength to pass. The excitation light blocking filter unit 40 is used together with the plurality of white light emitting units 21 and the plurality of excitation light emitting units 22 which are staggered with each other. After the fluorescence observation is completed, the fluorescent microscopic imaging apparatus 1 may immediately switch to use visible light as the light source in the same field of view for microscopic observation, thereby achieving the purpose of convenient to use and accurate judgment. It will not cause confusion and misjudgment in the image comparison due to the replacement of the light source, thereby achieving the purpose of convenient to use and accurate to judgment.

**Claims**

1. A fluorescent microscopic imaging apparatus (1) for detecting a skin to be tested, **characterized in that** the fluorescent microscopic imaging apparatus (1) comprising:

   a light guiding structure (10) having a first plane (11) and a second plane (12), the first plane (11) contacts the skin to be tested;
   a light emitting module (20) disposed on the second plane (12), the light emitting module (20) including a plurality of white light emitting units (21) and a plurality of excitation light emitting units (22) staggered with each other;
   an imaging module (30) disposed on the second plane (12) and disposed at a central position of the light emitting module (20); and
   an excitation light blocking filter unit (40) disposed between the first plane (11) and the imaging module (30), the excitation light blocking filter unit (40) blocking an excitation light with a specific wavelength emitted by each excitation light emitting unit (22).

2. The fluorescent microscopic imaging apparatus (1) in claim 1, wherein the distance between the imaging module (30) and the first plane (11) is L, the distance between the imaging module (30) and one side of each white light emitting unit (21) is R, the distance between the imaging module (30) and one side of each excitation light emitting unit (22) is R, the minimum angle between a normal line of the first plane (11) and an extension line of one side of each white light emitting unit (21) is a, the minimum angle between the normal line of the first plane (11) and an extension line of one side of each excitation light emitting unit (22) is a, and the foregoing content satisfies the following relationship:

$$\tan a = \frac{R}{L}, a \geq 30°.$$

3. The fluorescent microscopic imaging apparatus (1) in claim 1, wherein the light guiding structure (10) is a frustum of a cone, an annular surface (13) that is opaque and is formed between one side of the first plane (11) and one side of the second plane (12), and an area of the first plane (11) is smaller than an area of the second plane (12).

4. The fluorescent microscopic imaging apparatus (1) in claim 3, wherein the annular surface (13) is covered with a reflective film (131).

5. The fluorescent microscopic imaging apparatus (1) in claim 4, wherein the reflective film (131) is an aluminum metal film.

6. The fluorescent microscopic imaging apparatus (1) in claim 1, wherein the specific wavelength is between 350 nm and 400 nm.

7. The fluorescent microscopic imaging apparatus (1) in claim 1, wherein the specific wavelength is 365 nm.

8. The fluorescent microscopic imaging apparatus (1) in claim 1, wherein the excitation light blocking filter unit (40) is covered with the imaging module (30).

9. The fluorescent microscopic imaging apparatus (1) in claim 1, wherein the excitation light blocking filter unit (40) does not contact the imaging module (30).

10. The fluorescent microscopic imaging apparatus (1) in claim 1, wherein the excitation light blocking filter unit (40) blocks light having a wavelength between 300 nm and 400 nm.

11. The fluorescent microscopic imaging apparatus (1) in claim 1, wherein the first plane (11) is a flat glass (110) covered with an anti-reflection film (111), the anti-reflection film (111) provides a transmittance of light having a wavelength between 350 nm and 700 nm that is greater than 97%.

12. The fluorescent microscopic imaging apparatus (1) in claim 1, wherein the first plane (11) is a flat glass (110) covered with an oil-repellent hydrophobic film (112).

**13.** The fluorescent microscopic imaging apparatus (1) in claim 1, wherein the imaging module (30) includes a microscope lens (31), a photosensitive element (32) and a control circuit board (33), the microscope lens (31) is disposed in the second plane (12), and the microscope lens (31) is disposed between the excitation light blocking filter unit (40) and the photosensitive element (32), the photosensitive element (32) and the control circuit board (33) are disposed outside the second plane (12), and the photosensitive element (32) is aligned with the microscope lens (31) and coupled to the control circuit board (33).

**14.** The fluorescent microscopic imaging apparatus (1) in claim 13, wherein a distance between the microscope lens (31) and each white light emitting unit (21) is equal to a distance between the microscope lens and each excitation light emitting unit (22).

**15.** The fluorescent microscopic imaging apparatus (1) in claim 1, wherein the plurality of white light emitting units (21) and the plurality of excitation light emitting units (22) are arranged in a ring shape, the plurality of white light emitting units (21) do not emit light simultaneously with the plurality of excitation light emitting units (22).

**Amended claims in accordance with Rule 137(2) EPC.**

**1.** A fluorescent microscopic imaging apparatus (1) for detecting a skin to be tested, **characterized in that** the fluorescent microscopic imaging apparatus (1) comprising:

a light guiding structure (10) having a first plane (11) and a second plane (12), the first plane (11) contacts the skin to be tested;
a light emitting module (20) disposed on the second plane (12), the light emitting module (20) including a plurality of white light emitting units (21) and a plurality of excitation light emitting units (22) staggered with each other;
an imaging module (30) disposed under the second plane (12) and partially protruded above the second plane (12), the imaging module (30) disposed at a central position of the light emitting module (20); and
an excitation light blocking filter unit (40) disposed between the first plane (11) and the imaging module (30), the excitation light blocking filter unit (40) blocking an excitation light with a specific wavelength emitted by each excitation light emitting unit (22),
wherein, the distance between the imaging module (30) and the first plane (11) is L, the distance between the imaging module (30) and one side of each white light emitting unit (21) is R, the distance between the imaging module (30) and one side of each excitation light emitting unit (22) is R, the minimum angle between a normal line of the first plane (11) and an extension line of one side of each white light emitting unit (21) is $a$, the minimum angle between the normal line of the first plane (11) and an extension line of one side of each excitation light emitting unit (22) is $a$, and the foregoing content satisfies the following relationship: $\tan a = \dfrac{R}{L}$ , $a \geq 30°$.

**2.** The fluorescent microscopic imaging apparatus (1) in claim 1, wherein the light guiding structure (10) is a frustum of a cone, an annular surface (13) that is opaque and is formed between one side of the first plane (11) and one side of the second plane (12), and an area of the first plane (11) is smaller than an area of the second plane (12).

**3.** The fluorescent microscopic imaging apparatus (1) in claim 2, wherein the annular surface (13) is covered with a reflective film (131).

**4.** The fluorescent microscopic imaging apparatus (1) in claim 3, wherein the reflective film (131) is an aluminum metal film.

**5.** The fluorescent microscopic imaging apparatus (1) in claim 1, wherein the specific wavelength is between 350 nm and 400 nm.

**6.** The fluorescent microscopic imaging apparatus (1) in claim 1, wherein the specific wavelength is 365 nm.

**7.** The fluorescent microscopic imaging apparatus (1) in claim 1, wherein the excitation light blocking filter unit (40) is covered with the imaging module (30).

**8.** The fluorescent microscopic imaging apparatus (1) in claim 1, wherein the excitation light blocking filter unit (40) does not contact the imaging module (30).

**9.** The fluorescent microscopic imaging apparatus (1) in claim 1, wherein the excitation light blocking filter unit (40) blocks light having a wavelength between 300 nm and 400 nm.

**10.** The fluorescent microscopic imaging apparatus (1) in claim 1, wherein the first plane (11) is a flat glass (110) covered with an anti-reflection film (111), the anti-reflection film (111) provides a transmittance of light having a wavelength between 350 nm and 700 nm that is greater than 97%.

**11.** The fluorescent microscopic imaging apparatus (1) in claim 1, wherein the first plane (11) is a flat glass (110) covered with an oil-repellent hydrophobic film (112).

12. The fluorescent microscopic imaging apparatus (1) in claim 1, wherein the imaging module (30) includes a microscope lens (31), a photosensitive element (32) and a control circuit board (33), the microscope lens (31) is disposed in the second plane (12), and the microscope lens (31) is disposed between the excitation light blocking filter unit (40) and the photosensitive element (32), the photosensitive element (32) and the control circuit board (33) are disposed outside the second plane (12), and the photosensitive element (32) is aligned with the microscope lens (31) and coupled to the control circuit board (33).

13. The fluorescent microscopic imaging apparatus (1) in claim 12, wherein a distance between the microscope lens (31) and each white light emitting unit (21) is equal to a distance between the microscope lens and each excitation light emitting unit (22).

14. The fluorescent microscopic imaging apparatus (1) in claim 1, wherein the plurality of white light emitting units (21) and the plurality of excitation light emitting units (22) are arranged in a ring shape, the plurality of white light emitting units (21) do not emit light simultaneously with the plurality of excitation light emitting units (22).

11

10

1

100

# FIG.1

FIG.2A

FIG.2B

<u>1</u>

FIG.3

32       33       50

| photosensitive element |—| control circuit board |—| transmission interface |

21 —| white light emitting units |

22 —| excitation light emitting units |

# FIG.4

Europäisches
Patentamt

European
Patent Office

Office européen
des brevets

# EUROPEAN SEARCH REPORT

Application Number

EP 19 19 9038

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | US 2016/241757 A1 (CHENG CHU-MING [TW] ET AL) 18 August 2016 (2016-08-18) | 1,2,6-15 | INV. G02B21/00 G01N21/64 G02B21/08 |
| Y | * abstract; figures 1-7 * * line 8, paragraph 7 * * paragraph [0028] - paragraph [0034] * ----- | 3-5 | |
| Y | EP 2 206 460 A1 (ANMO ELECTRONICS CORP [TW]) 14 July 2010 (2010-07-14) | 3-5 | |
| A | * abstract; figures 8-11 * ----- | 1,2,6-15 | |
| A | EP 1 830 305 A1 (FUJITSU LTD [JP]; FUJITSU FRONTECH LTD [JP]) 5 September 2007 (2007-09-05) * abstract; figures 1,9 * ----- | 1-15 | |

TECHNICAL FIELDS
SEARCHED      (IPC)

G02B
G01N

The present search report has been drawn up for all claims

| Place of search | Date of completion of the search | Examiner |
|---|---|---|
| Munich | 16 April 2020 | Jakober, François |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 9038

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2020

| Patent document cited in search report | | | Publication date | Patent family member(s) | | | Publication date |
|---|---|---|---|---|---|---|---|
| US 2016241757 | A1 | | 18-08-2016 | CN | 105877699 | A | 24-08-2016 |
| | | | | TW | 201628548 | A | 16-08-2016 |
| | | | | US | 2016241757 | A1 | 18-08-2016 |
| EP 2206460 | A1 | | 14-07-2010 | CN | 101766468 | A | 07-07-2010 |
| | | | | CN | 201775617 | U | 30-03-2011 |
| | | | | EP | 2206460 | A1 | 14-07-2010 |
| | | | | TW | M383115 | U | 21-06-2010 |
| | | | | TW | 201027984 | A | 16-07-2010 |
| | | | | US | 2010171827 | A1 | 08-07-2010 |
| EP 1830305 | A1 | | 05-09-2007 | CN | 101030245 | A | 05-09-2007 |
| | | | | EP | 1830305 | A1 | 05-09-2007 |
| | | | | JP | 4745084 | B2 | 10-08-2011 |
| | | | | JP | 2007229360 | A | 13-09-2007 |
| | | | | KR | 20070090725 | A | 06-09-2007 |
| | | | | US | 2007206098 | A1 | 06-09-2007 |

EPO FORM P0459

For more details about this annex : see Official Journal of the European Patent Office, No. 12/82